(19) Europäisches Patentamt
European Patent Office
Office européen des brevets

(11) EP 0 968 998 A1

(12) DEMANDE DE BREVET EUROPEEN

(43) Date de publication:
**05.01.2000 Bulletin 2000/01**

(51) Int Cl.[7]: **C07C 233/19**, A61K 7/48

(21) Numéro de dépôt: **99401538.6**

(22) Date de dépôt: **21.06.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **03.07.1998 FR 9808563**

(71) Demandeur: **ELF ATOCHEM S.A.**
**92800 Puteaux, Hauts-de-Seine (FR)**

(72) Inventeurs:
- **Lagrange, Sophie**
**31520 Ramonville-Saint-Agne (FR)**
- **Fraisse, Laurent**
**31130 Balma (FR)**

# (54) Compositions cosmétiques, nouveaux analogues de céramides à activité anti-élastase, procédé de préparation et utilisations

(57) L'invention se rapporte à l'amélioration des propriétés anti-élastase de compositions cosmétiques par incorporation de composés de formule :

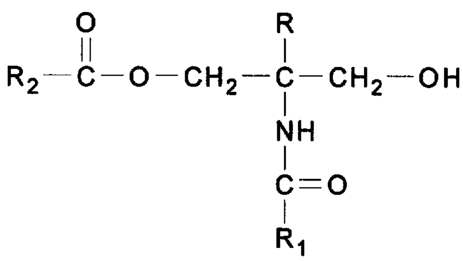

(I)

dans laquelle

R représente un atome d'hydrogène, un radical alkyle en $C_1$-$C_4$ ou un radical hydroxyalkyle en $C_1$-$C_4$ ;
$R_1$ et $R_2$, identiques ou différents, représentent chacun une chaîne hydrocarbonée linéaire ou ramifiée en $C_4$-$C_{40}$ pouvant porter un ou plusieurs groupes hydroxyles, une ou plusieurs insaturations ou renfermer une ou plusieurs fonctions ester, éther ou amide.

Elle concerne également de nouveaux analogues de céramides, leur procédé de préparation et leur utilisation.

EP 0 968 998 A1

## Description

La présente invention se rapporte au domaine des cosmétiques, et concerne plus particulièrement l'incorporation d'analogues de céramides dans les compositions cosmétiques, dermocosmétiques ou pharmaceutiques en vue d'améliorer leurs propriétés anti-élastase.

La peau humaine contient de l'élastine, une protéine fibreuse qui, en association avec des glycoprotéines, forme les fibres élastiques responsables de l'élasticité des tissus.

La production d'élastine commence dès le stade embryonnaire et se poursuit jusqu'à l'âge de 20 ans environ. Dès lors débute le processus de vieillissement normal des tissus qui tend à réduire leur élasticité. Il se produit une diminution et un ralentissement de la synthèse de l'élastine, d'une part, et une augmentation de la teneur en élastases qui hydrolysent l'élastine, d'autre part. Il en résulte une raréfaction des fibres élastiques, la peau perd sa cohésion, devient flasque et des rides apparaissent en surface.

Des facteurs externes tels que l'exposition chronique aux rayons solaires accélèrent le vieillissement cutané. Les UV notamment induisent un épaississement et un raccourcissement des fibres élastiques qui n'assurent plus leur rôle dans la structure de la peau et activent les élastases.

Plusieurs méthodes ont été proposées pour atténuer les rides apparaissant lors du vieillissement cutané.

On a envisagé d'incorporer de l'élastine dans des compositions cosmétiques afin que cette dernière constitue un substrat des élastases de la peau. L'application de telles compositions a certes pour effet de ralentir la dégradation de l'élastine endogène. Elle s'accompagne toutefois d'une augmentation de la teneur en fragments d'élastine dans la partie supérieure de l'épiderme (le stratum corneum), ce qui entraîne une kératinisation excessive.

On a proposé d'atténuer les rides en appliquant sur la peau des agents favorisant la desquamation des cellules mortes. L'apparence lisse obtenue par cette méthode est temporaire.

On a encore décrit des compositions à base d'un gel de polymère hydrophile dont le rôle est de combler les rides et les ridules et qui, en séchant, confèrent à la peau un aspect lisse et satiné.

On a enfin proposé d'agir sur le processus de vieillissement de la peau en bloquant l'action des élastases. Les élastases sont synthétisées par les fibroblastes cutanés et les leucocytes, notamment les leucocytes polynucléaires. Ces enzymes sont capables de dégrader l'élastine et d'autres constituants de la peau. Elles renferment un site de fixation de l'élastine et un site assurant la coupure. Les inhibiteurs des élastases peuvent se fixer sur l'un ou l'autre site.

Les inhibiteurs d'élastases connus dans le domaine cosmétique sont soit naturels, principalement sous forme d'extraits végétaux (voir par exemple JP-A-10045555; JP-A-10036281 ; FR 2 746 316 ; FR 2 727 313 et V. BIZOT-FOULON et al., International Journal of Cosmetic Science, Vol. 17, pp. 255-264 (1995) soit synthétiques (voir par exemple US 5 614 489 et FR 2 746 316).

Les inhibiteurs qui présentent une analogie structurale avec les céramides naturels de la peau s'avèrent particulièrement intéressants.

On sait en effet que la peau est riche en céramides, en particulier au niveau des couches supérieures de l'épiderme.

Les céramides forment une classe particulière de lipides caractérisés en ce qu'ils sont constitués de sphingosine (ou d'un dérivé de cette dernière) amidifiée par un acide gras. Ils jouent un rôle prépondérant dans le maintien de la cohésion du stratum corneum et la régulation de la perte d'eau transépidermique. En outre, ils sont connus pour avoir un effet de barrière à l'encontre des agressions extérieures, notamment par protection des fibres élastiques avec lesquelles ils ont une forte affinité.

Les inhibiteurs d'élastases qui présentent une forte analogie de structure avec les céramides naturels sont susceptibles de pénétrer facilement et rapidement dans le derme et, par conséquent, d'agir efficacement sur les élastases. De tels inhibiteurs d'élastases analogues de céramides sont décrits dans la demande de brevet WO 96/11956. Ils sont obtenus par greffage de chondroïtine sulfate sur un céramide et sont plus spécialement adaptés pour combattre le vieillissement cutané photo-induit.

Le besoin est donc grand de disposer de nouvelles compositions renfermant des inhibiteurs d'élastase analogues de céramides qui ont la faculté d'améliorer les propriétés de la barrière cutanée et qui, en outre, peuvent être préparées d'une façon aisée et relativement peu coûteuse.

Il a maintenant été trouvé que l'on peut améliorer les propriétés anti-élastase de compositions cosmétiques, dermocosmétiques ou pharmaceutiques en leur incorporant au moins un analogue de céramide répondant à la formule générale :

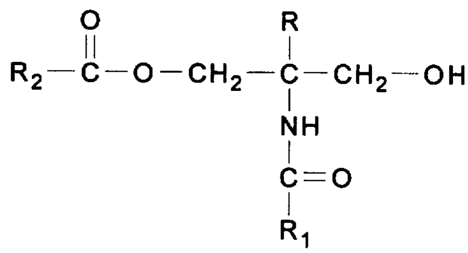

(I)

dans laquelle

R représente un atome d'hydrogène, un radical alkyle en $C_1$-$C_4$ ou un radical hydroxyalkyle en $C_1$-$C_4$ ;
$R_1$ et $R_2$, identiques ou différents, représentent chacun une chaîne hydrocarbonée linéaire ou ramifiée en $C_4$-$C_{40}$ pouvant porter un ou plusieurs groupes hydroxyles, une ou plusieurs insaturations ou renfermer une ou plusieurs fonctions ester, éther ou amide.

Les composés conformes à l'invention contiennent de préférence des groupes $R_1$ et $R_2$ renfermant 14 à 30 atomes de carbone, et mieux encore 16 à 24 atomes de carbone.
Conformément à la présente invention, on peut utiliser un seul composé de formule (I) ou un mélange de ces composés.
D'une façon générale, les composés de formule (I) peuvent être préparés selon le procédé qui consiste :

a- à faire réagir un amino-glycol de formule :

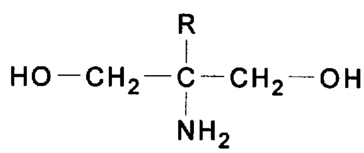

(II)

dans laquelle R a la signification donnée précédemment,
et un agent acylant de formule :

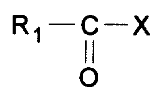

(III)

dans laquelle $R_1$ a la signification donnée précédemment et X représente un halogène, de préférence le chlore, ou un radical $OR_3$ dans lequel $R_3$ représente un atome d'hydrogène, un radical aliphatique, linéaire ou ramifié, saturé ou insaturé, en $C_1$-$C_6$ ou un radical acyle en $C_2$-$C_{41}$,
pour former le composé de formule :

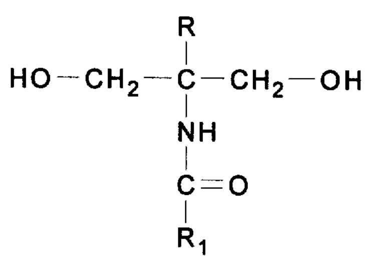

(IV)

dans laquelle R et $R_1$ ont la signification donnée précédemment, et
b- à faire réagir le produit de réaction de l'étape a- et un agent acylant de formule :

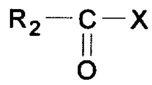

(V)

dans laquelle $R_2$ et X ont la signification donnée précédemment,

pour former le composé de formule :

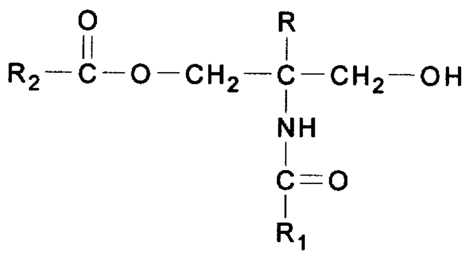

(I)

dans laquelle R, $R_1$ et $R_2$ ont la signification donnée précédemment.

La préparation des composés de formule (I) dans lesquels $R_1$ et $R_2$ sont identiques est avantageusement mise en oeuvre selon une variante du procédé précité qui consiste à opérer dans les conditions de l'étape a- seulement.

L'amino-glycol de formule (II) est choisi dans le groupe constitué par le 2-amino-1,3-propanediol, le 2-amino-2-méthyl-1,3-propanediol, le 2-amino-2-éthyl-1,3-propanediol et le 2-amino-2-hydroxyméthyl-1,3-propanediol.

A titre d'exemples non limitatifs de composés de formule (III) ou (V) utilisables dans l'une ou l'autre des variantes du procédé, on peut citer les chlorures d'acyle tels que le chlorure de palmitoyle, le chlorure de stéaroyle et le chlorure d'oléoyle, les acides carboxyliques tels que l'acide palmitique, l'acide stéarique et l'acide oléique, les esters d'acide carboxyliques tels que les esters méthylique ou éthylique des acides carboxyliques précités, et les anhydrides d'acide tels que l'anhydride stéarique et acétique. On utilise de préférence les chlorures d'acyle et les esters d'acides carboxyliques.

La préparation des composés de formule (I) conformes à l'invention peut être mise en oeuvre par fusion des réactifs de formule (II), (III) et (V) ou en présence d'un solvant.

Tout solvant peut être utilisé dans le procédé pour autant qu'il n'est pas susceptible de réagir avec l'un quelconque des réactifs mis en jeu. A titre d'exemples de tels solvants, on peut citer les hydrocarbures aliphatiques tels que l'hexane ou l'heptane, les hydrocarbures cycloaliphatiques tels que le cyclohexane, les hydrocarbures aromatiques tels que le toluène, les composés hétérocyliques oxygénés tels que le tétrahydrofurane ou azotés tels que la pyridine, et les amines tertiaires telles que la triéthylamine. Il est possible d'utiliser l'eau mais seulement lors de l'étape a-.

De préférence le solvant est anhydre.

Lorsque les composés de formule (III) et/ou (V) sont un acide carboxylique ou un ester d'acide carboxylique, on opère en présence d'un catalyseur chimique ou enzymatique.

A titre d'exemples de catalyseurs chimiques, on peut citer les acides tels que l'acide sulfurique, l'acide méthanesulfonique ou l'acide para-toluènesulfonique, et les bases telles que l'hydroxyde de sodium, l'hydroxyde de potassium ou le méthylate de sodium.

A titre d'exemples de catalyseurs enzymatiques, on peut citer les hydrolases telles que les estérases, plus particulièrement les lipases telles que les lipases produites par *Mucor miehei, Candida rugosa, Candida antarctica* ou *Rhizopus arrhizus,* et les protéases. De tels catalyseurs peuvent se présenter sous la forme d'enzymes libres ou immobilisées sur un support solide minéral ou organique. Ces catalyseurs peuvent être mis en oeuvre dans un réacteur du type colonne ou fonctionnant par lot ("batch" en anglais).

Dans la mise en oeuvre du procédé, le rapport molaire du composé (II) au composé de formule (III) ou (V) est généralement compris entre 10:1 et 1:10, et de préférence 2:1 et 1:2.

On effectue en général la réaction à une température pouvant varier de 10 à 150°C, de préférence de 40 à 80°C, et sous agitation.

Il est possible d'opérer sous une pression comprise entre 0,5 kPa et 100 kPa, de préférence 2 kPa et 100 kPa, et sous un courant de gaz inerte tel que l'azote ou l'argon.

La durée de la réaction peut varier dans une large mesure. Elle est le plus souvent comprise entre 2 et 30 heures, et de préférence 4 et 24 heures.

A l'issue de la réaction, le milieu peut éventuellement subir un lavage et/ou une ou plusieurs purifications

Le lavage peut être effectué au moyen d'eau ou d'une solution acide diluée, par exemple de l'ordre de 5 %.

A titre d'exemple de purification, on peut citer la recristallisation, de préférence à chaud, dans un solvant approprié, par exemple un alcool tel que l'éthanol ou le méthyl-2-propanol-2, un solvant chloré tel que le chloroforme ou le dichlorométhane, et un mélange chloroforme/hexane, ou encore la chromatographie sur une colonne remplie de silice et élution avec un solvant chloré tel que le chloroforme ou le dichlorométhane, un mélange binaire d'un de ces solvants et de méthanol, et un mélange d'acétate d'éthyle et d'hexane ou de cyclohexane.

Parmi les composés de formule (I) selon l'invention, on préfère ceux dans lesquels R représente un radical alkyle en $C_2$-$C_4$ ou un radical hydroxyalkyle en $C_1$-$C_4$, et plus particulièrement ceux dans lesquels R est un radical éthyle ou hydroxyméthyle.

La quantité d'analogue de céramide de formule (I) à introduire dans une composition cosmétique, dermocosmétique ou pharmaceutique pour obtenir l'efficacité anti-élastase recherchée est comprise entre 0,001 et 20 % par rapport au poids de la composition, de préférence comprise entre 0,1 et 10 %.

La composition cosmétique, dermocosmétique ou pharmaceutique peut être une lotion, un lait, une crème, un gel, un baume, un shampooing, un après shampooing, un produit de traitement du cuir chevelu ou des ongles ou un produit de maquillage. La composition peut se présenter sous la forme d'une huile, d'une émulsion, d'une dispersion aqueuse ou non, d'une pâte ou d'une poudre.

La composition comprend au moins un véhicule aidant la pénétration de l'analogue de céramide dans la peau.

A titre d'exemples de véhicules, on peut citer :

- les émollients tels que les acides gras, les alcools gras et les huiles,
- les solvants tels que l'éthanol, l'isopropanol, le propylèneglycol, le butylèneglycol et le polyéthylèneglycol 400,
- les humectants tels que la glycérine, la gélatine et le sorbitol,
- les adsorbants tels que la cellulose micronisée, les bentonites, les silicates, la craie, le talc, l'amidon, la gomme, les polyacrylates et les polyamides, notamment du type ORGASOL® commercialisé par ELF ATOCHEM, et
- les agents de solubilisation tels que les esters de polyglycéryle.

Le véhicule représente généralement 80 à 99,9 % du poids total de la composition, et de préférence 90 à 99,5 %.

La composition peut contenir, en outre, des agents susceptibles d'interagir avec la peau et favoriser la pénétration de l'analogue de céramide.

Parmi ces agents, on peut citer les composés qui provoquent une diminution de la résistance naturelle de la peau tels que les solvants, les agents de desquamation (par exemple les alpha hydroxyacides) et les tensio-actifs neutres (par exemple un alcool ou un acide gras éthoxylé) ou anionique (par exemple les savons, les N-acyl aminoacides, les acyltaurates, les alkyl éther carboxylates, les alkyl phosphates, les alkyl éther phosphates et les alkyllactates.

Ces agents peuvent aussi être des vecteurs de pénétration de l'analogue précité tels que les microcapsules ou des nanocapsules (par exemple des liposomes et des niosomes ou des composés favorisant la constitution de phases lamellaires.

La composition peut également renfermer des composés biologiquement actifs tels que des agents hydratants (par exemple de l'acide hyaluronique ou des dextranes sulfates).

Les analogues de céramides selon l'invention peuvent être utilisés soit en remplacement des céramides ou des analogues de céramides à activité anti-élastase dans les compositions cosmétiques, dermocosmétiques ou pharmaceutiques existantes, soit en sur-addition dans ces compositions.

Ces analogues sont en outre susceptibles d'avoir une action sur la régulation de la perte d'eau transépidermique.

Les exemples qui suivent permettent d'illustrer l'invention. Dans ces exemples, on utilise les méthodes d'analyse suivantes :

- Spectrographie infrarouge (IR)

  Les spectres sont réalisés au moyen d'un appareil PERKIN ELMER 1760 IRTF, en dispersion dans KBr à 1 %. Les valeurs sont exprimées en nombre d'onde par centimètre.
- Résonance magnétique nucléaire (RMN)

  Les spectres sont réalisés au moyen d'un appareil BRUCKER DRX 500, à 500 MHz ($^1H$) et 125 MHz ($^{13}C$) dans le DMSO-$d_6$. La calibration de l'appareil est effectuée au moyen de DMSO. Les déplacements chimiques sont exprimés en ppm. L'aspect des signaux est noté : singulet (s), doublet (d), triplet (t), quadruplet (q), multiplet (m), quintuplet (*5) et couplage AB (AB).
- Mesure de l'activité anti-élastase

  L'activité anti-élastase est mesurée selon la méthode qui consiste à faire réagir l'élastase de leucocytes humains (référence SE 563 ; Elastin Products Co.; Owensville, MO, USA) avec le N-succinyl-$(Ala)_3$-p-nitroanilide (réf. S4760 ; Sigma) et mesurer la libération de p-nitroaniline.

On utilise le N-succinyl-(Ala)3-p-nitroanilide en solution dans le tétrahydrofurane et une solution d'élastase à 100 mg/ml dans un mélange de glycérol et de tampon acétate de sodium (50/50 ; v/v), pH5.

L'activité de l'enzyme est mesurée à 37°C dans 100 mM de tampon Tris-HCI, pH8 contenant 0,01 % de Triton X-100 (volume/volume) et 0,02 % d'azide de sodium (poids/volume). Le céramide à tester, solubilisé dans le tétrahydrofurane (concentration en THF = 0,5 % en volume) est préincubé en présence de l'enzyme (0,05 mg/ml) dans le tampon pendant 5 minutes à 37°C. On ajoute le N-succinyl-$(Ala)_3$-p-nitroanilide (concentration finale : 250 µM) et on mesure la libération de la p-nitroaniline dans une cuve en quartz par spectrophotométrie à 410 nm.

La capacité inhibitrice du céramide est exprimée en pourcentage d'inhibition (% I) selon la relation :

$$\%I = (1 - \frac{Vi}{Vo}) \times 100$$

dans laquelle Vi et Vo sont les vitesses d'apparition de la p-nitroaniline en présence et en absence d'inhibiteur respectivement.

- Mesure de l'amélioration de la barrière cutanée

*a)* mesure de la Perte Insensible en Eau (PIE)

La mesure de la PIE permet de quantifier la très faible quantité d'eau qui diffuse passivement à travers le *stratum corneum.* Ce paramètre représente un très bon index de la fonction barrière du *stratum corneum.*

Cette méthode est surtout utilisée pour contrôler l'efficacité de formules cosmétiques qui, contenant certains types de lipides sont capables de diminuer la PIE et, en conséquence, d'empêcher la déshydratation du *stratum corneum.*

La sonde est laissée en contact de la peau pendant 2 minutes environ de façon à atteindre une position d'équilibre.

$$PIE = D' \frac{dP}{dx} \text{ (gradient de pression de vapeur)}$$

Les valeurs sont exprimées en g/$m^2$/heure et permettent de calculer un delta de PIE = PIE (peau traitée) - PIE (peau témoin).

L'appareil utilisé est un Evaporimètre EPI de Servomed.

Les mesures sont effectuées sur deux groupes de 15 volontaires sains ayant une peau sèche, avant l'application du produit (t=0) et après l'application (t=0,5 heure, 1 heure, 3 heures et 6 heures) sur la face interne des deux avant-bras. L'analogue de céramide selon l'invention ou le céramide témoin est incorporé dans une crème ayant la composition suivante (en % en poids) :

| | |
|---|---|
| Alcool gras saturé éthoxylé (Cremophor A6 ; BASF) | 3,25 |
| Alcool gras saturé éthoxylé (Cremophor A25 ; BASF) | 1,75 |
| Triglycéride caprilique/caprique (Miglyol 812 N ; HULS) | 3,00 |
| Cire d'abeille stéarique (Ester BW 67 ; KOSTER) | 1,00 |
| Pentastéarate de polyglycérol-10 (Décaglyn 5-S ; NIKKO) | 5,00 |
| Alcool cétylique (Nacol 16/98 ; CONDEA) | 4,00 |
| Huile d'avocat vierge (BERTIN) | 2,00 |
| Stéarate d'octyle (Cetiol 868 ; HENKEL) | 2,00 |
| Dioctylcyclohexane (Cetiol S; HENKEL) | 3,00 |
| Esters (phényle/méthyle/butyle/ éthyle/propyle) d'acide parahydroxybenzoïque (Phenonip ; SIPCA) | 0,50 |
| Eau déminéralisée qsp 100 | |

La teneur en analogue de céramide dans la crème est égale à 0,5 % (exemple 7), 1 % (exemples 4 et 8) et 5 % (exemple 6), et celle des céramides témoins (a) et (b) est égale à 0,5 %.

La diminution des valeurs de PIE signifie une amélioration de la qualité de la barrière cutanée qui traduit un meilleur pouvoir retentionnel de l'eau au niveau de la couche cornée et donc une meilleure hydratation.

*b)* hydratation cutanée

L'eau peut être considérée comme un dipôle électrique capable de conduire l'électricité. Le transport d'électricité dans un champ donné est proportionnel à la quantité d'eau présente dans le *stratum corneum.* Plus une peau est riche en eau et plus le courant électrique passe rapidement entre 2 électrodes.

Il existe donc un lien entre l'hydratation du *stratum corneum* et les modifications des propriétés électriques.

Les mesures de capacitance, liée à la constante diélectrique de la peau, sont réalisées par le Cornéomètre Courage+Khazaka CM 200, dont la sonde présente une surface de contact avec la peau de 49 $mm^2$ ; cette sonde est appliquée avec une pression constante sur la surface cutanée à mesurer, et l'indice d'hydratation directement proportionnel au taux hydrique de surface est instantané.

Sur la zone de mesure (avant-bras - voir paragraphe a) ci-avant), 3 mesures sont effectuées en triangle et rapprochées, dont la moyenne est automatiquement calculée et représente l'Indice d'Hydratation (IH) individuel.

Les indices d'hydratation individuels sont moyennés sur l'ensemble des sujets.

## EXEMPLE 1

Dans un ballon maintenu à 50°C et sous un courant d'azote, on introduit 0,9 g (10 mmol) de 2-amino-1,3-propanediol (Réf. 35789-8 ; ALDRICH) dissous dans 20 ml de chloroforme contenant 5,6 ml (40 mmol) de triéthylamine. A la solution ainsi obtenue, on ajoute goutte à goutte en 1,5 heure, une solution contenant 6,8 ml (22 mmol) de chlorure de palmitoyle (Réf. P7-8; ALDRICH) dans 20 ml de chloroforme.

Après 2 heures à 50°C, le milieu réactionnel est porté au reflux pendant 1 heure. Le milieu est lavé avec une solution aqueuse d'acide chlorhydrique à 5 % en poids et de l'eau. La phase organique est récupérée et évaporée à sec. Le résidu obtenu est recristallisé dans un mélange chloroforme/hexane (8/2 (v/v)) et purifié sur une colonne remplie de silice (éluant: chloroforme/méthanol 95/5 (v/v)). On obtient le 1-palmitoyl-2-palmitoylamino-1,3-propanediol avec un rendement molaire de 30 %.

Ce composé présente les caractéristiques suivantes :

IR : 1547 et 1641 (amide), 1725 (ester)

RMN:

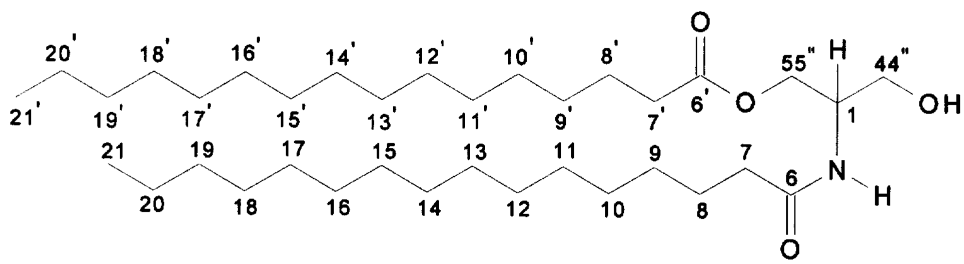

$^1H$ : H5' : 4,14 (m); H5" : 3,98 (m); H1 : 3,98 (m); H4 et H4":3,44 et 3,38 (2m) ; H7':2,25(t); H7 : 2,07 (td) ; H8': 1,54 (*5) ; H8 : 1,49 (*5) ; H9 à H20 : 1,43 à 1,10 (m); H9' à H20': 1,43 à 1,10; H21 + H21': 0,87 (t); NH : 7,32 (d); OH : 4,52 (t).

$^{13}C$ : C6 et C6' : 172,3 et 171,8; C5 : 62,7 ou 60,3; C4 : 60,3 ou 62,7; C1 : 49,5 ; C7: 35,2; C7':33,3; C19 + C19': 30,9 ; C9 à C18 : 28,6 à 28,1 ; C9' à C18':28,6 à 28,1; C8:24,9; C8':24,1; C2:23,3; C20 + C20':21,6; C21 + C21':13,3.

Activité anti-élastase

Le tableau 1 rassemble les valeurs du pourcentage d'inhibition (%I) pour des concentrations en 1-palmitoyl-2-palmitoylamino-1,3-propanediol égales à 250 μM et 500 μM.

## EXEMPLE 2

Dans un ballon équipé d'un dispositif d'agitation et maintenu à 55°C, on introduit 1,15 g (11 mmol) de 2-amino-2-méthyl-1,3-propanediol (ANGUS) solubilisé dans 10 ml d'eau contenant 4,7 ml (33 mmol) de triéthylamine et une solution contenant 3 ml (9,7 mmol) de chlorure de palmitoyle (Réf. P7-8 ; ALDRICH) dans 10 ml de tétrahydrofurane. Après 24 heures de réaction à 55°C, le solvant est évaporé et le résidu obtenu est dissous dans du chloroforme et lavé avec une solution aqueuse d'acide chlorhydrique à 5 % en poids et de l'eau.

Le produit obtenu est recristallisé deux fois dans du chloroforme à 60°C. On obtient 2 g de 2-méthyl-2-palmitoylamino-1,3-propanediol.

Dans un ballon maintenu à 55°C et sous un courant d'azote, on introduit 0,92 g (2,7 mmol) du produit ainsi obtenu dissous dans 75 ml de chloroforme anhydre contenant 0,87 ml (6,2 mmol) de triéthylamine. On introduit ensuite,

goutte à goutte en 90 minutes, une solution contenant 0,88 ml (2,8 mmol) de chlorure de palmitoyle (Réf. P7-8; ALDRICH) dans 15 ml de chloroforme anhydre. Le milieu réactionnel est porté au reflux pendant 3 heures puis est lavé avec une solution aqueuse d'acide chlorhydrique à 5 % en poids et de l'eau. Le solvant est évaporé à sec et le résidu obtenu est recristallisé dans un mélange chloroforme/hexane (8/2 (v/v)) à 4°C pendant 24 heures. Après purification sur une colonne remplie de silice (éluant: cyclohexane/acétate d'éthyle; 70/30 (v/v)), on récupère le 1-palmitoyl-2-méthyl-2-palmitoylamino-1,3-propanediol avec un rendement molaire global de 20 %.

Activité anti-élastase

Le tableau 1 rassemble les valeurs du pourcentage d'inhibition (%I) pour des concentrations en 1-palmitoyl-2-méthyl-2-palmitoylamino-1,3-propanediol égales à 250 µM et 500 µM.

**EXEMPLE 3**

Dans un ballon maintenu à 45°C et sous un courant d'azote, on introduit 5 g (42 mmol) de 2-amino-2-éthyl-1,3-propanediol (ANGUS) dissous dans 100 ml de chloroforme anhydre contenant 31 ml (227 mmol) de triéthylamine. A la solution ainsi obtenue, on ajoute goutte à goutte en 3 heures, une solution contenant 13 ml (42 mmol) de chlorure de palmitoyle (Ref. P7-8 ; ALDRICH) dans 100 ml de chloroforme. Après 30 minutes d'agitation, on effectue un nouvel ajout de chlorure de palmitoyle dans les conditions précitées.

Le milieu réactionnel est porté au reflux pendant 20 heures, puis il est lavé avec une solution aqueuse d'acide chlorhydrique à 5 % et un mélange eau/chlorure de sodium à saturation. La phase organique est récupérée et évaporée à sec. Le résidu obtenu est recristallisé dans un mélange chloroforme/hexane (80/20 (v/v)) à 60°C et purifié sur une colonne remplie de silice (éluant: cyclohexane/acétate d'éthyle 70/30 (v/v)). On obtient le 1-palmitoyl-2-éthyl-2-palmitoylamino-1,3-propanediol avec un rendement molaire de 50 %.

Ce composé présente les caractéristiques suivantes :

RMN:

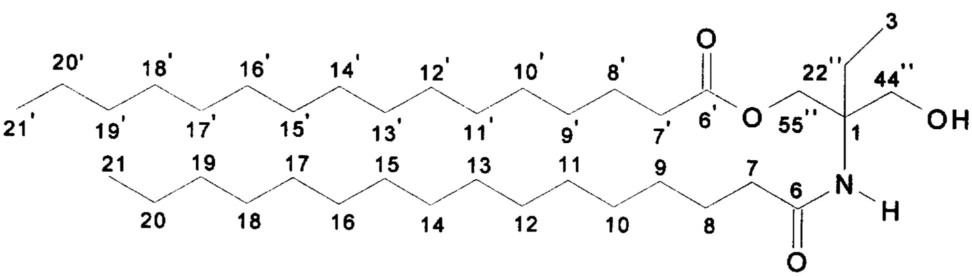

$^{1}H$ : H5' et H5'' : 4,21 et 4,16 (AB) ; H4 et H4'' : 3,55 et 3,46 (AB) ; H7' : 2,26 (t) ; H7 : 2,07 (t) ; H2 : 1,74 (m) ; H2'' : 1,63 (m) ; H8' : 1,54 (*5) ; H8 : 1,49 (*5) ; H9 à H20 : 1,40 à 1,10 (m) ; H9'à H20' : 1,40 à 1,10 (m) ; H21 + H21': 0,27 (t); H3 : 0,80 (t).

$^{13}C$ : C6 et C6' : 172,4 et 172,2; C5 : 63,3; C4 : 61,6; C1 : 58,7; C7 : 35,8; C7' : 33,4 : C19 + C19' : 30,8; C9 à C18 : 28,6 à 28,1 ; C9'à C18' : 28,6 à 28,1 ; C8 : 25,0 ; C8' : 24,1 ; C2 : 23,3 ; C20 + C20': 21,6; C21 + C21': 13,3; C3 : 6,8.

Activité anti-élastase

Le tableau 1 rassemble les valeurs du pourcentage d'inhibition (% I) pour des concentrations en 1-palmitoyl-2-éthyl-2-palmitoylamino-1,3-propanediol égales à 250 µM et 500 µM.

**EXEMPLE 4**

Dans un ballon maintenu à 60°C et équipé d'un dispositif d'agitation (155 tours/min) et d'un régulateur de vide relié à une trompe à eau, on introduit 1 g (8,4 mmol) de 2-amino-2-éthyl-1,3-propanediol (ANGUS) et 4,65 g (15,1 mmol) de stéarate de méthyle (Réf. M8,070-9 ; ALDRICH).

Lorsque les produits sont fondus, on introduit 0,5 mmol de méthylate de sodium (soit 0,06 équivalent par rapport au dérivé propanediol engagé) en trois fois sur une période de 7 heures, et on diminue la pression à 40 kPa, puis à 10 kPa.

Après 7 heures de réaction, on porte la pression à 2 kPa pendant 1 heure afin d'éliminer le dérivé propanediol résiduel. Le résidu obtenu est recristallisé à 60°C dans un mélange d'éthanol (30 ml) et d'acide chlorhydrique à 36 % (42,5 µl soit 0,5 mmol).

Activité anti-élastase

Le tableau 1 rassemble les valeurs du pourcentage d'inhibition (%I) pour des concentrations en 1-stéaroyl-2-éthyl-2-stéaroylamino-1,3-propanediol égales à 250 pM et 500 µM.

Mesure de l'amélioration de la barrière cutanée

Les mesures de perte insensible en eau et d'hydratation cutanée sont rassemblées dans les tableaux 2 et 3 respectivement.

## EXEMPLE 5

On procède dans les conditions de l'exemple 2, première étape, modifié en ce que l'on utilise 1,3 g (11 mmol) de 2-amino-2-éthyl-1,3-propanediol (ANGUS) et que l'on effectue une seule recristallisation. On obtient le 2-éthyl-2-palmitoylamino-1,3-propanediol avec un rendement molaire de 30 %.

Dans un ballon, on introduit 0,357 g (1 mmol) de 2-éthyl-2-palmitoylamino-1,3-propanediol, 0,3 g (1 mmol) d'oléate de méthyle (Ref. 0-4754 ; SIGMA), 150 g de lipase supportée (Chirazym L2 ; Boehringer) et 10 ml d'octane. Après 8 heures de réaction à 90°C, on récupère le 1-oléoyl-2-éthyl-2-palmitoylamino-1,3-propanediol avec un rendement molaire global de 15 %.

## EXEMPLE 6

Dans un ballon maintenu à 65°C et équipé d'un dispositif d'agitation et d'un régulateur de vide, on introduit 1 g (8,4 mmol) de 2-amino-2-éthyl-1,3-propanediol (ANGUS), 4,5 g (15,1 mmol) d'oléate de méthyle (ALDRICH) et on amène la pression à 10 kPa. Après une heure, on introduit rapidement 128 µl (0,672 mmol) de méthylate de sodium. Après 24 heures de réaction, on forme le 1-oléoyl-2-éthyl-2-oléoylamino-1,3-propanediol avec un rendement de 47 %.

Ce dernier est purifié par chromatographie sur une colonne remplie de silice (éluant : hexane/acétate d'éthyle 80/20 puis 40/60 (v/v)).

Les mesures de l'amélioration de la barrière cutanée sont données dans les tableaux 2 (perte insensible en eau) et 3 (hydratation cutanée).

## EXEMPLE 7

On procède dans les conditions de l'exemple 4 modifié en ce que l'on utilise 1 g (9,5 mmol) de 2-amino-2-méthyl-1,3-propanediol (ANGUS) et 5,26 g (17,8 mmol) de stéarate de méthyle.

Après 23 heures de réaction, on obtient le 1-stéaroyl-2-méthyl-2-stéaroylamino-1,3-propanediol avec un rendement de 72 %.

Les mesures de l'amélioration de la barrière cutanée sont données dans les tableaux 2 (perte insensible en eau) et 3 (hydratation cutanée).

## EXEMPLE 8

On chauffe à 140°C une suspension de tris(hydrométhyl)amino-méthane (500 mg ; 4,1 mol) dans du stéarate de méthyle (2,52 g ; 8,2 mmol) à laquelle on ajoute 16 µl (82 µmol) de méthylate de sodium (un fort dégagement de méthanol est observé). Après une heure, on diminue la température du milieu réactionnel à 110°C et on ajoute par deux fois 16 µl de méthylate de sodium.

Après 2 heures, le chauffage est arrêté et le milieu réactionnel refroidi est neutralisé avec une solution d'acide chlorhydrique 1M, et cristallisé successivement dans l'heptane et l'éthanol.

On obtient 1 g de 1-stéaroyl-2-hydroxyméthyl-2-stéaroylamino-1,3-propanediol (rendement: 38 %).

Les mesures de l'amélioration de la barrière cutanée sont données dans les tableaux 2 (perte insensible en eau) et 3 (hydratation cutanée).

TABLEAU 1

| Exemple | Concentration (μM) | % I (%) |
|---|---|---|
| 1 | 250 | 55 |
| | 500 | 66 |
| 2 | 250 | 8 |
| | 500 | 57 |
| 3 | 250 | 33 |
| | 500 | 43 |
| 4 | 250 | 67 |
| | 500 | 86 |
| Référence [a] | 250 | 81 |
| | 500 | 100 |
| Référence [b] | 250 | 44 |
| | 500 | 50 |

(a) N-palmitoyl-D-érythro-1,3-dihydroxy-2-amino-4-trans octadécène (Céramide naturel préparé par synthèse chimique ; Bio Lab Ltd)
(b) N-stéaroyl-phytosphingosine (céramide naturel préparé par fermentation et synthèse chimique ; Gist Brocades).

## TABLEAU 2

| EXEMPLE | | TEMPS (heure) | | | | |
|---|---|---|---|---|---|---|
| | | 0 | 0,5 | 1 | 3 | 6 |
| 4 | M±s | 4,52±0,79 | 3,89±0,85 | 2,95±1,15 | 4,30±0,97 | 4,00±1,24 |
| | % évol. | | - 14 % | - 35 % | - 5 % | - 11 % |
| 6 | M±S | 3,96±1,60 | 3,67±1,34 | 3,48±1,19 | 3,39±1,20 | 3,97±1,20 |
| | % évol. | | - 7 % | - 12 % | - 15 % | 0 % |
| 7 | M±s | 4,50±2,0 | 4,07±1,72 | 3,78±1,67 | 3,70±1,50 | 4,46±1,64 |
| | % évol. | | - 10 % | - 16 % | - 18 % | - 1 % |
| 8 | M±s | 4,52±1,02 | 4,28±1,38 | 3,12±1,11 | 4,62±1,14 | 4,55±1,39 |
| | % évol. | | - 5 % | - 31 % | + 2 % | + 1 % |
| Témoin (a) | M±s | 4,40±0,90 | 3,91±1,40 | 3,37±1,00 | 4,20±1,09 | 4,11±1,41 |
| | % évol. | | - 11 % | - 23 % | - 4 % | - 7 % |
| Témoin (b) | M±s | 4,22±2,17 | 4,09±1,86 | 3,80±1,75 | 3,53±1,57 | 4,36±1,82 |
| | % évol. | | - 3 % | - 10 % | - 16 % | + 3 % |

(a) : N-stéaroyl-D,L-sphingosine (Sederma)
(b) : N-stéaroyl phytosphingosine (Cosmoferm)

## TABLEAU 3

| EXEMPLE | | TEMPS (heure) | | | | |
|---|---|---|---|---|---|---|
| | | 0 | 0,5 | 1 | 3 | 6 |
| 4 | M±s | 64,69±9,04 | 66,87±7,50 | 65,56±6,96 | 62,31±7,17 | 62,91±7,78 |
| | % évol. | | + 3 % | + 1 % | - 4 % | - 3 % |
| 6 | M±S | 61,91±6,90 | 78,53±6,23 | 75,29±7,78 | 67,05±5,61 | 64,56±6,14 |
| | % évol. | | + 27 % | + 22 % | + 8 % | + 4 % |
| 7 | M±s | 60,13±6,27 | 66,13±5,48 | 65,56±6,24 | 63,82±7,05 | 61,55±6,32 |
| | % évol. | | + 10 % | + 9 % | + 6 % | + 2 % |
| 8 | M±s | 64,13±8,01 | 67,16±7,98 | 67,04±7,74 | 64,13±8,37 | 64,87±7,83 |
| | % évol. | | + 5 % | + 5 % | 0 % | + 1 % |
| Témoin (a) | M±s | 63,95±8,45 | 67,11±7,74 | 67,93±9,08 | 62,67±6,59 | 63,47±6,46 |
| | % évol. | | + 5 % | + 6 % | - 2 % | - 1 % |
| Témoin (b) | M±s | 61,00±7,21 | 67,55±7,89 | 67,45±7,33 | 63,20±5,36 | 61,36±3,79 |
| | % évol. | | + 11 % | + 11 % | + 4 % | + 1 % |

(a) : N-stéaroyl-D,L-sphingosine (Sederma)
(b) : N-stéaroyl phytosphingosine (Cosmoferm)

**Revendications**

1. Compositions cosmétiques, dermocosmétiques ou pharmaceutiques, caractérisées en ce qu'elles contiennent :

   - au moins un analogue de céramide de formule :

   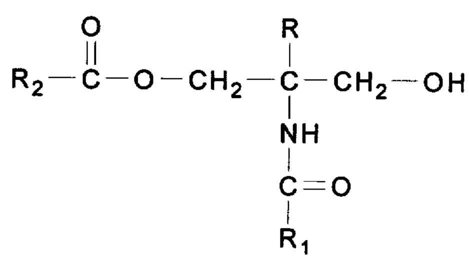

   (I)

   dans laquelle

   R représente un atome d'hydrogène, un radical alkyle en $C_1$-$C_4$ ou un radical hydroxyalkyle en $C_1$-$C_4$ ;
   $R_1$ et $R_2$, identiques ou différents, représentent chacun une chaîne hydrocarbonée linéaire ou ramifiée en $C_4$-$C_{40}$ pouvant porter un ou plusieurs groupes hydroxyles, une ou plusieurs insaturations ou renfermer une ou plusieurs fonctions ester, éther ou amide,

   - et au moins un véhicule cosmétiquement, dermocosmétiquement ou pharmaceutiquement acceptable.

2. Compositions selon la revendication 1, dans lesquelles $R_1$ et $R_2$ représentent des chaînes hydrocarbonées en $C_{14}$-$C_{30}$.

**3.** Compositions selon la revendication 2, dans lesquelles $R_1$ et $R_2$ représentent des chaînes hydrocarbonées en $C_{16}$-$C_{24}$.

**4.** Compositions selon l'une des revendications 1 à 3, dans lesquelles R représente un radical alkyle en $C_2$-$C_4$ ou un radical hydroxyalkyle en $C_1$-$C_4$.

**5.** Compositions selon l'une des revendications 1 à 4, dans lesquelles R est un radical éthyle ou hydroxyméthyle.

**6.** Compositions selon l'une des revendications 1 à 5, dans lesquelles la teneur en analogue(s) de céramide(s) est comprise entre 0,001 et 20 % en poids de la composition.

**7.** Analogues de céramides de formule :

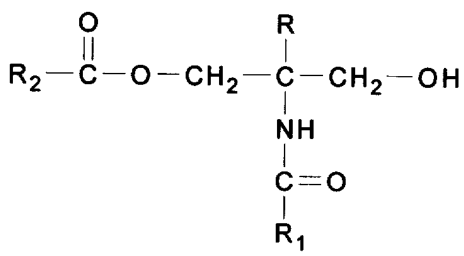

(I)

dans laquelle

R représente un radical alkyle en $C_2$-$C_4$ ou un radical hydroxyalkyle en $C_1$-$C_4$ ;
$R_1$ et $R_2$, identiques ou différents, représentent chacun une chaîne hydrocarbonée linéaire ou ramifiée en $C_4$-$C_{40}$ pouvant porter un ou plusieurs groupes hydroxyles, une ou plusieurs insaturations ou renfermer une ou plusieurs fonctions ester, éther ou amide.

**8.** Analogues selon la revendication 7, dans lesquels R représente un radical éthyle ou hydroxyméthyle.

**9.** Analogues selon la revendication 7 ou 8, dans lesquels $R_1$ et $R_2$ sont des chaînes hydrocarbonées en $C_{14}$-$C_{30}$.

**10.** Analogues selon la revendication 9, dans lesquels $R_1$ et $R_2$ sont des chaînes hydrocarbonées en $C_{16}$-$C_{24}$.

**11.** Procédé de préparation des composés selon l'une des revendications 7 à 10, qui consiste :

a- à faire réagir un amino-glycol de formule :

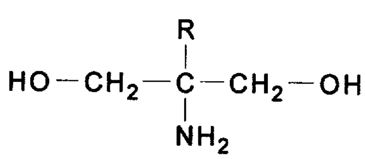

(II)

dans laquelle R a la signification donnée précédemment,
et un agent acylant de formule :

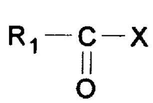

(III)

dans laquelle $R_1$ a la signification donnée précédemment et X représente un halogène, de préférence le chlore, ou un radical $OR_3$ dans lequel $R_3$ représente un atome d'hydrogène, un radical aliphatique, linéaire ou ramifié, saturé ou insaturé, en C1-C6 ou un radical acyle en $C_2$-$C_{41}$,

pour former le composé de formule :

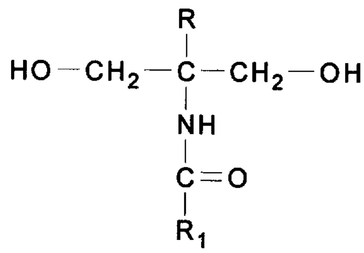

(IV)

dans laquelle R et $R_1$ ont la signification donnée précédemment, et

b- à faire réagir le produit de réaction de l'étape a- et un agent acylant de formule :

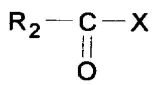

(V)

dans laquelle $R_2$ et X ont la signification donnée précédemment,

pour former le composé de formule :

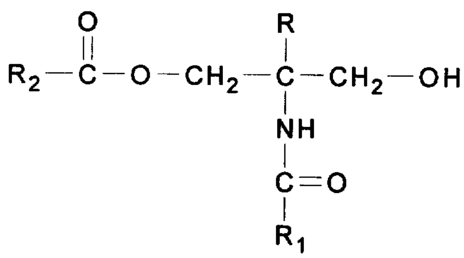

(I)

dans laquelle R, $R_1$ et $R_2$ ont la signification donnée précédemment.

**12.** Procédé selon la revendication 11, dans lequel les réactifs sont mis en oeuvre par fusion ou par solubilisation dans un solvant organique approprié.

**13.** Procédé selon l'une des revendications 11 ou 12, dans lequel l'agent d'acylation est un chlorure d'acyle.

**14.** Procédé selon l'une des revendications 11 ou 12, dans lequel on utilise un agent acylant choisi parmi les acides carboxyliques et les esters d'acides carboxyliques, et un catalyseur.

**15.** Procédé selon la revendication 14, dans lequel le catalyseur est un catalyseur chimique choisi parmi les acides et les bases.

**16.** Procédé selon la revendication 14, dans lequel le catalyseur est un catalyseur enzymatique choisi parmi les hydrolases telles que les estérases et les protéases.

**17.** Utilisation des compositions selon la revendication 1 pour le traitement non thérapeutique de la peau, du cuir chevelu et des ongles.

**18.** Utilisation des analogues selon la revendication 7 pour la préparation de compositions à usage cosmétique, dermocosmétique ou pharmaceutique.

Office européen des brevets

# RAPPORT DE RECHERCHE EUROPEENNE

Numéro de la demande
EP 99 40 1538

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A,D | WO 96 11956 A (FABRE PIERRE DERMO COSMETIQUE) 25 avril 1996 (1996-04-25)<br>* page 2 - page 3; revendications * | 1,11,17 | C07C233/19<br>A61K7/48 |
| A | EP 0 253 489 A (CURTIS HELENE IND INC) 20 janvier 1988 (1988-01-20)<br>* page 13 - page 16; revendications * | 1,11,17 | |
| A | FR 2 425 442 A (NATTERMANN A & CIE) 7 décembre 1979 (1979-12-07)<br>* revendications * | 1,11,17 | |
| A | RANSAC, S. ET AL: "Stereoselectivity of lipases. I. Hydrolysis of enantiomeric glyceride analogs by gastric and pancreatic lipases, a kinetic study using the monomolecular film technique"<br>J. BIOL. CHEM. (1990), 265(33), 20263-70 CODEN: JBCHA3;ISSN: 0021-9258, XP002100366<br>* page 20264 * | 1,11,17 | |
| A | GANONG, BARRY R. ET AL: "Specificity and mechanism of protein kinase C activation by sn-1,2-diacylglycerols"<br>PROC. NATL. ACAD. SCI. U. S. A. (1986), 83(5), 1184-8 CODEN: PNASA6;ISSN: 0027-8424, XP002100367<br>* page 1185 * | 1,11,17 | DOMAINES TECHNIQUES RECHERCHES (Int.Cl.7)<br>C07C<br>A61K |

Le présent rapport a été établi pour toutes les revendications

| Lieu de la recherche | Date d'achèvement de la recherche | Examinateur |
|---|---|---|
| LA HAYE | 23 septembre 1999 | Sánchez García, J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 99 40 1538

La présente annexe indique les membres de la famille de brevets relatifs aux documents brevets cités dans le rapport de recherche européenne visé ci–dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

23-09-1999

| Document brevet cité au rapport de recherche | Date de publication | Membre(s) de la famille de brevet(s) | Date de publication |
|---|---|---|---|
| WO 9611956 A | 25-04-1996 | FR 2725622 A | 19-04-1996 |
| | | AT 168381 T | 15-08-1998 |
| | | DE 69503514 D | 20-08-1998 |
| | | EP 0785952 A | 30-07-1997 |
| EP 0253489 A | 20-01-1988 | AT 86472 T | 15-03-1993 |
| | | AU 597668 B | 07-06-1990 |
| | | AU 7376987 A | 17-12-1987 |
| | | CA 1295949 A | 18-02-1992 |
| | | DE 3784571 A | 15-04-1993 |
| | | DK 303287 A | 17-12-1987 |
| | | ES 2053538 T | 01-08-1994 |
| | | FI 872668 A,B, | 17-12-1987 |
| | | JP 7088291 B | 27-09-1995 |
| | | JP 63066112 A | 24-03-1988 |
| | | NZ 220644 A | 27-09-1989 |
| | | PT 85078 A | 01-07-1988 |
| | | US 4830774 A | 16-05-1989 |
| | | US 5154847 A | 13-10-1992 |
| FR 2425442 A | 07-12-1979 | DE 2820893 A | 22-11-1979 |
| | | CA 1117965 A | 09-02-1982 |
| | | GB 2020663 A,B | 21-11-1979 |
| | | JP 54148727 A | 21-11-1979 |
| | | US 4221732 A | 09-09-1980 |
| | | ZA 7902284 A | 25-06-1980 |

EPO FORM P0460

Pour tout renseignement concernant cette annexe : voir Journal Officiel de l'Office européen des brevets, No.12/82